# EUROPEAN PATENT APPLICATION

(11) **EP 4 813 321 A1**
(43) Date of publication of application: **30.09.2026**
(21) Application number: 24949291.9
(22) Date of filing: 22.08.2024
(51) Int. Cl.: A61B 17/221, A61B 17/3207

(54) **THROMBECTOMY DEVICE**

(30) Priority: 02.08.2024 CN 202411056325
(71) Applicant: Shenzhen Betterway Medtech Co., Ltd., Shenzhen, Guangdong 518000 (CN)
(72) Inventor: WU, Xingyu, Shenzhen, Guangdong 518000 (CN); LI, Zhenjie, Shenzhen, Guangdong 518000 (CN); LONG, Han, Shenzhen, Guangdong 518000 (CN)
(74) Representative: Bayramoglu et al.
(86) International application number: PCT/CN2024/113881
(87) International publication number: WO 2026/025566

(57) **Abstract**

Disclosed is a thrombectomy device (1), which comprises a cutting stent (10) with an inner cavity, wherein the cutting stent (10) is a grid structure composed of a plurality of grid units (12), and each grid unit (12) comprises a plurality of support rods (121) connected end to end; the cutting stent (10) comprises a cutting part (11) located at the proximal end, and the cutting stent (10) comprises a first extension region (14), a second extension region (15), a third extension region (16), and a fourth extension region (17) which are extending in the axial direction; the first extension region (14), the second extension region (15), the third extension region (16), and the fourth extension region (17) are sequentially arranged and connected in the circumferential direction, and the cutting part (11) is connected to the proximal ends of the first extension region (14), the second extension region (15), the third extension region (16), and the fourth extension region (17); a distal end portion (111) of the cutting part (11) is adjacent to a proximal end portion of the first extension region (14), and a proximal end portion (112) of the cutting part (11) is adjacent to a proximal end portion of the third extension region (16); the hardness of the first extension region (14) is greater than the hardness of the second extension region (15) and the fourth extension region (17), and the hardness of the third extension region (16) is greater than the hardness of the second extension region (15) and the fourth extension region (17). The cutting part (11) of the thrombectomy device (1) is not easily separated from the blood vessel wall.

## Description

### TECHNICAL FIELD

The present disclosure relates to the technical field of medical apparatus, and in particular to a thrombectomy device.

### BACKGROUND

The information provided in this part is merely related to the background art of the present disclosure and does not necessarily constitute the prior art.

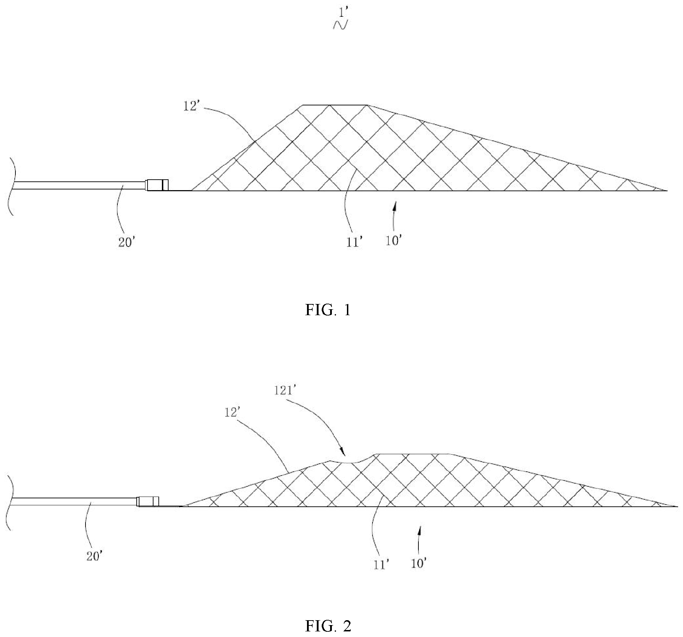

Referring to FIG. 1, a thrombectomy device 1' known to the applicant includes a thrombectomy stent 10' and a pulling tube 20'.

The thrombectomy stent 10' includes a plurality of end-to-end mesh rods 11', and defines an inner cavity. An opening portion 12' communicating with the inner cavity is formed at a proximal end of the thrombectomy stent 10', and the opening portion 12' is an oblique opening (instead of a flat opening perpendicular to the axial direction). A distal end of the pulling tube 20' is connected to the proximal end of the thrombectomy stent 10'. During thrombectomy, the thrombectomy stent 10' is delivered to a distal side of a thrombus, and the pulling tube 20' is driven by a pulling force to move the thrombectomy stent 10' proximally in a blood vessel. The opening portion 12' of the thrombectomy stent 10' can strip the thrombus on the inner wall of the blood vessel from the blood vessel wall, so that the thrombus is captured in the inner cavity of the thrombectomy stent 10' and the captured thrombus is carried out of the patient's body by the thrombectomy stent 10'.

In some applications, the inner diameter of the blood vessel gradually decreases along the moving direction of the thrombectomy stent 10', and the opening portion 12' is an oblique opening. Therefore, when the thrombectomy stent 10' enters a blood vessel with a smaller inner diameter from a blood vessel with a larger inner diameter, a proximal portion of the opening portion 12' enters the blood vessel with a smaller inner diameter earlier than a distal portion thereof, which easily causes excessive concentration of the mesh rods 11' at the distal end portion of the opening portion 12'. Further, the mesh rods 11' near the distal end portion are folded inward under the action of a radially inward force applied by the inner wall of the blood vessel, forming a recess 121' at the distal end portion as shown in FIG. 2. As a result, the opening portion 12' is separated from the inner wall of the blood vessel at the recess 121', leading to low thrombectomy efficiency.

### SUMMARY

Based on this, it is necessary to provide a thrombectomy device whose opening portion is not easily separated from the blood vessel wall.

A thrombectomy device includes a cutting stent having an inner cavity. The cutting stent is a grid structure with a plurality of grid units. Each grid unit includes a plurality of end-to-end support rods. The cutting stent includes a cutting portion at a proximal end. The cutting portion is an opening communicating with the inner cavity. The cutting portion includes a proximal end portion and a distal end portion. The cutting stent includes a first extension region, a second extension region, a third extension region, and a fourth extension region extending in an axial direction. The first extension region, the second extension region, the third extension region, and the fourth extension region are sequentially arranged and connected in a circumferential direction. The cutting portion is connected to proximal ends of the first extension region, the second extension region, the third extension region, and the fourth extension region. A distal end portion of the cutting portion is adjacent to a proximal end portion of the first extension region. A proximal end portion of the cutting portion is adjacent to a proximal end portion of the third extension region. The first extension region and the third extension region are radially opposite. The second extension region and the fourth extension region are radially opposite. A hardness of the first extension region is greater than a hardness of the second extension region. A hardness of the first extension region is greater than a hardness of the fourth extension region. A hardness of the third extension region is greater than a hardness of the second extension region. A hardness of the third extension region is greater than a hardness of the fourth extension region.

The above thrombectomy device includes the cutting stent, and the cutting stent includes the first extension region, the second extension region, the third extension region, and the fourth extension region extending in an axial direction. The first extension region, the second extension region, the third extension region, and the fourth extension region are sequentially arranged and connected in a circumferential direction. The first extension region is radially opposite to the third extension region, and the second extension region is radially opposite to the fourth extension region. The distal end portion of the cutting portion is adjacent to the proximal end portion of the first extension region, and the proximal end portion of the cutting portion is adjacent to the proximal end portion of the third extension region. In the thrombectomy device provided by the embodiment of the present disclosure, by setting the hardness of the first extension region to be greater than the hardness of the second extension region, and setting the hardness of the first extension region to be greater than the hardness of the fourth extension region, so that during the process of the cutting stent gradually entering a blood vessel with a smaller inner diameter, the support rods in the second extension region and the fourth extension region undergo a larger circumferential shrinkage deformation than the support rods in the first extension region, thereby reducing or avoiding excessive accumulation of the support rods in the first extension region. This prevents excessive accumulation of the support rods near the distal end portion of the cutting portion, and can reduce or avoid inward folding of the support rods near the distal end portion of the cutting portion due to excessive accumulation, so as to improve the wall adherence of the distal end portion of the cutting portion in the blood vessel. In addition, the hardness of the third extension region is greater than the hardness of the second extension region and the hardness of the fourth extension region, so that the third extension region has a relatively high hardness, thereby reducing the axial stretching amplitude during thrombectomy, and further reducing the inward shrinkage deformation amplitude caused by axial stretching of the cutting portion, so as to improve the wall adherence of the cutting portion. Therefore, the thrombectomy device provided by the embodiment of the present disclosure has good wall adherence.

### BRIEF DESCRIPTION OF THE DRAWINGS

To clearly illustrate the technical solutions in the embodiments of the present disclosure, the accompanying drawings required for describing the embodiments will be briefly introduced below. Obviously, the drawings described below are merely some embodiments of the present disclosure, and those skilled in the art can obtain other drawings based on these drawings without creative efforts.

Wherein:
FIG. 1 is a schematic structural view of a known thrombectomy device provided by the applicant;
FIG. 2 is a state view of inward recessing of a distal end portion of an opening portion of a known thrombectomy stent;
FIG. 3 is a perspective view of a thrombectomy device according to an embodiment;
FIG. 4 is an exploded perspective view of a thrombectomy device according to an embodiment;
FIG. 5 is a schematic structural view of a thrombectomy device according to an embodiment;
FIG. 6 is a top view of FIG. 5;
FIG. 7A is a plan view of a first stent according to an embodiment, which is cut along an axial direction of the cutting stent from a distal end portion of the cutting portion and unfolded into a planar state;
FIG. 7B is a plan view of the first extension region of the first stent shown in FIG. 7A unfolded into a planar state;
FIG. 7C is a plan view of the second extension region and the third extension region of the first stent shown in FIG. 7A unfolded into a planar state;
FIG. 7D is a plan view of the fourth extension region of the first stent shown in FIG. 7A unfolded into a planar state;
FIG. 7E is a plan view of the cutting portion of the first stent shown in FIG. 7A unfolded into a planar state; and
FIG. 8 is a plan view of a part of the first stent in FIG. 7A unfolded into a planar state.

### DETAILED DESCRIPTION OF THE EMBODIMENTS

The technical solutions in the embodiments of the present disclosure will be clearly and completely described below with reference to the accompanying drawings in the embodiments of the present disclosure. Obviously, the described embodiments are merely a part of the embodiments of the present disclosure, rather than all of the embodiments. Based on the embodiments of the present disclosure, all other embodiments obtained by those skilled in the art without creative efforts shall fall within the protection scope of the present disclosure.

In the description of the embodiments of the present disclosure, it should be noted that orientation or positional relationships indicated by terms such as "center", "upper", "lower", "left", "right", "vertical", "horizontal", "inner" and "outer" are based on the orientation or positional relationships shown in the accompanying drawings, which are only for the convenience of describing the embodiments of the present disclosure and simplifying the description, and do not indicate or imply that the referred apparatus or element must have a specific orientation, be constructed and operated in a specific orientation, and thus should not be construed as limiting the embodiments of the present disclosure. In addition, terms "first", "second" and "third" are used for descriptive purposes only and should not be construed as indicating or implying relative importance.

In the description of the embodiments of the present disclosure, it should be noted that unless otherwise clearly specified and limited, terms "mounted", "connected" and "linked" should be understood in a broad sense. For example, they may be fixed connections, replaceable connections, or integral connections; they may be mechanical connections or electrical connections; they may be direct connections or indirect connections through an intermediate medium; they may be internal communication between two elements. For those skilled in the art, the specific meanings of the above terms in the embodiments of the present disclosure can be understood according to specific situations.

In the field of interventional medical apparatuss, the end of a medical apparatus implanted in a human or animal body that is relatively close to an operator is generally referred to as a "proximal end", and the end that is relatively far from the operator is referred to as a "distal end". The "proximal end" and "distal end" of any component of the medical apparatus are defined according to this principle. "Axial direction" generally refers to the length direction of the medical apparatus during delivery, and "radial direction" generally refers to a direction of the medical apparatus that is not parallel to the "axial direction". The "axial direction" and "radial direction" of any component of the medical apparatus are defined according to this principle. "Circumferential direction" refers to a circumferential direction, i.e., a direction surrounding the axis of a tubular structure or a cylinder.

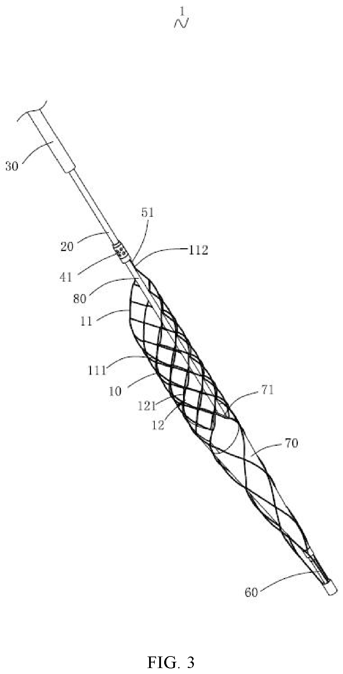

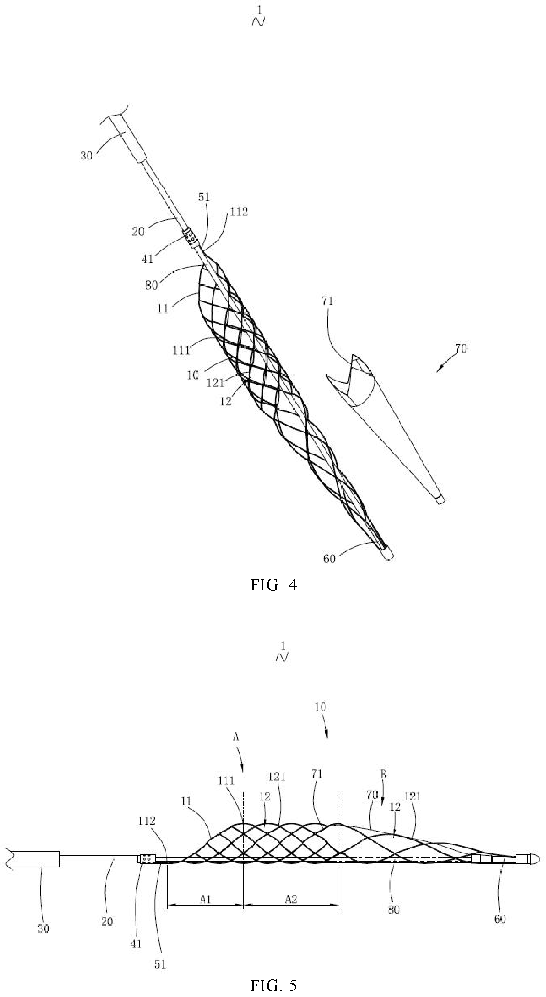

Referring to FIGS. 3 and 4, the present disclosure provides a thrombectomy device 1, which can be used to remove a thrombus in a blood vessel of a patient. The thrombectomy device 1 includes a cutting stent 10, a push-pull tube 20, and an outer tube 30. A distal end of the push-pull tube 20 is connected to a proximal end of the cutting stent 10. The outer tube 30 is slidably sleeved on the push-pull tube 20. The outer tube 30 can slide axially along the push-pull tube 20 to load and release the cutting stent 10.

The cutting stent 10 is a grid structure having an inner cavity. The grid structure includes a plurality of grid units 12. each grid unit 12 includes a plurality of end-to-end support rods 121. A hollow portion of each grid unit 12 is a mesh hole. The cutting stent 10 can be made by braiding braided wires and then heat-setting. The cutting stent 10 can also be made by cutting a hollow tube and then heat-setting. The braided wires can be nickel-titanium alloy wires, stainless steel wires, or other metal wires. The hollow tube can be a metal tube such as a nickel-titanium alloy tube or a stainless steel tube, or a polymer tube.

The cutting stent 10 has radial compressibility, and can be compressed to a loading size under a binding force (a radial compression force applied to the cutting stent 10 or an axial pulling force applied to both ends of the cutting stent 10), so that the cutting stent 10 can be loaded into the outer tube 30 and delivered in the blood vessel of the patient through the outer tube 30. After reaching a desired position, the outer tube 30 is operated to move proximally relative to the cutting stent 10 and the push-pull tube 20 to expose the cutting stent 10.

The cutting stent 10 has self-expansibility. When the binding force applied to the cutting stent 10 is removed, the cutting stent 10 can expand to a radially expanded state. For example, after the outer tube 30 sleeved on the cutting stent 10 is retracted to be separated from the cutting stent 10, the cutting stent 10 can radially self-expand to a radially expanded state.

Continuing to refer to FIGS. 3 and 4, a cutting portion 11 communicating with the inner cavity is formed at the proximal end of the cutting stent 10. The cutting portion 11 is a circumferentially closed structure, and can cut a thrombus when moving in a blood vessel under an external force.

Continuing to refer to FIGS. 3 and 4, the cutting portion 11 includes a distal end portion 111 and a proximal end portion 112. The distal end portion 111 is located at a distal side of the proximal end portion 112. When the cutting stent 10 is located in a blood vessel and the binding force applied to the cutting stent 10 is removed, the cutting stent 10 can self-expand to a radially expanded state, so that the cutting portion 11 is attached to the inner wall of the blood vessel.

Referring to FIGS. 3 and 4, the thrombectomy device 1 further includes a mounting ring 41. The mounting ring 41 is connected to the proximal end portion 112 of the cutting portion 11 through a connecting rod 51.

The push-pull tube 20 is a hollow tubular structure. A distal end of the push-pull tube 20 is connected to the mounting ring 41, so as to be connected to the proximal end portion 112 of the cutting portion 11 through the mounting ring 41 and the connecting rod 51, enabling the push-pull tube 20 to apply a force to the cutting stent 10, thereby pushing the cutting stent 10 to a distal side of a thrombus during thrombectomy. After the cutting stent 10 is delivered to a desired position (e.g., at a distal side of a thrombus), a pulling force is applied to the cutting stent 10 to move the cutting stent 10 in the blood vessel to scrape the thrombus.

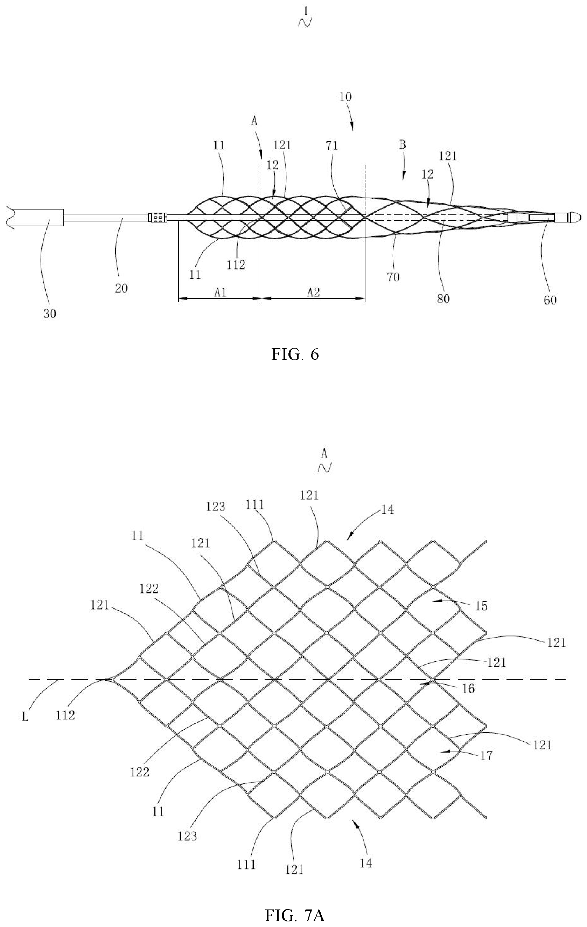

Referring to FIGS. 5 and 6 together, the cutting stent 10 axially includes a first stent A and a second stent B connected to each other. The first stent A is located at a proximal side of the second stent B, and both the first stent A and the second stent B are parts of the grid structure and include a plurality of grid units 12. A circumferentially closed edge at a proximal end of the first stent A constitutes the cutting portion 11. The first stent A and the second stent B are connected to each other and jointly define the inner cavity of the cutting stent 10.

Continuing to refer to FIGS. 5 and 6, the first stent A includes a first axial section A1 and a second axial section A2 connected in sequence from proximal to distal. It should be noted that, when the grid unit 12 is formed by a part of the cutting portion 11 together with other support rods 121, the part of the cutting portion 11 forming the grid unit 12 can also be referred to as a support rod 121.

Referring to FIGS. 5 and 6 together, the first axial section A1 is an axial section where the cutting portion 11 is located. An axial length of the first axial section A1 is equal to an axial length of the cutting portion 11. A proximal end of the second axial section A2 is connected to the first axial section A1.

Referring to FIGS. 6 and 7A together, when the first stent A is unfolded into a planar shape, the first axial section A1 is substantially in an isosceles triangle shape. The first axial section A1 includes a sharp corner portion at a proximal end and a wave portion at a distal end (not marked). The sharp corner portion is the proximal end portion 112 of the cutting portion 11, and distal end portions of two waists of the isosceles triangle form the distal end portion 111 of the cutting portion 11 when closed. It should be noted that the above "unfolded into a planar state" means that the cutting stent 10 is cut from the distal end portion 111 along the axial direction of the cutting stent 10 and unfolded into a planar state, and when the cutting stent 10 is in the "unfolded into a planar state", the first stent A is also necessarily in the "unfolded into a planar state".

Referring to FIGS. 6 and 7A together, the second axial section A2 is cylindrical and has a cylindrical inner cavity with openings at both ends. A distal end of the second axial section A2 is connected to a proximal end of the second stent B. An end of the second axial section A2 away from the second stent B is connected to the first axial section A1.

During operation of the thrombectomy device 1 for thrombectomy, the cutting portion 11 of the cutting stent 10 is first placed at a distal side of a thrombus, then the binding force applied to the cutting stent 10 is removed, and the cutting stent 10 self-expands, so that the cutting portion 11 is attached to the blood vessel wall. Since the cutting portion 11 is a circumferentially closed structure, the cutting portion 11 can be attached to the blood vessel wall in a 360° circumferential direction. A pulling force is applied to the push-pull tube 20 to drive the cutting stent 10 to move proximally, thereby cutting the thrombus on the blood vessel wall. The thrombus cut by the cutting portion 11 enters the inner cavity of the cutting stent 10 through the cutting portion 11 to be captured, and is then carried out of the body by the cutting stent 10.

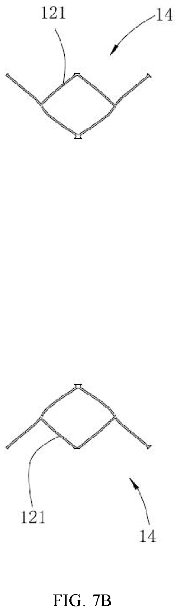

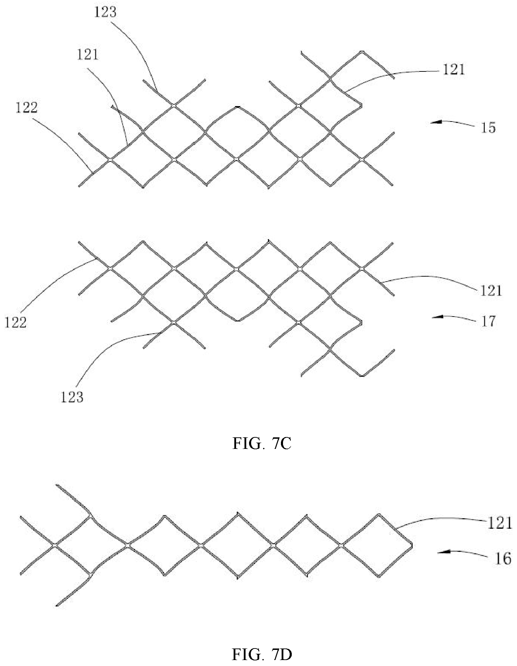

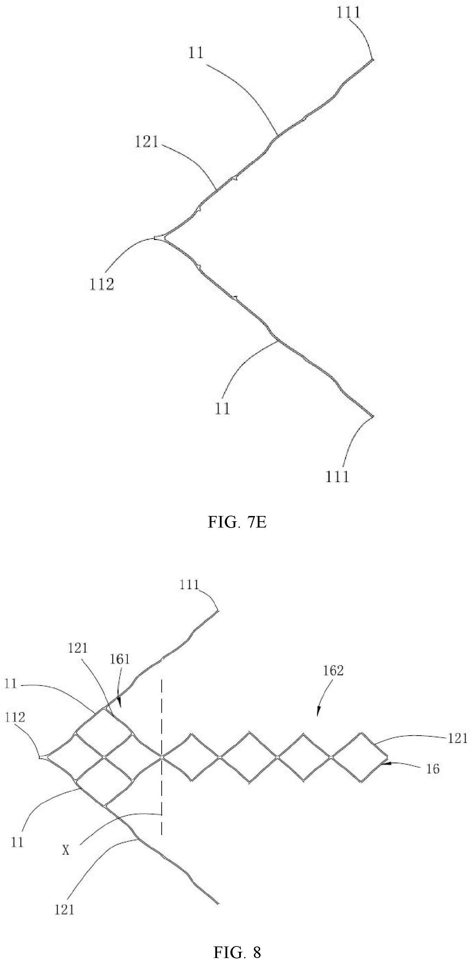

Referring to FIG. 7A together, the first stent A of this embodiment includes a first extension region 14 (refer to FIGS. 7A and 7B together for the first extension region 14), a second extension region 15 (refer to FIGS. 7A and 7C together for the second extension region 15), a third extension region 16 (refer to FIGS. 7A and 7D together for the third extension region 16), and a fourth extension region 17 (refer to FIGS. 7A and 7C together for the fourth extension region 17) extending in the axial direction. The first extension region 14, the second extension region 15, the third extension region 16, and the fourth extension region 17 are sequentially arranged and connected in the circumferential direction (the circumferential direction of the three-dimensional structure, not the planar unfolded view). The first extension region 14 is radially opposite to the third extension region 16. The second extension region 15 is radially opposite to the fourth extension region 17. The cutting portion 11 (refer to FIGS. 7A and 7E for a state of the cutting portion 11 unfolded into a planar state) is connected to proximal ends of the first extension region 14, the second extension region 15, the third extension region 16, and the fourth extension region 17. The first extension region 14, the second extension region 15, the third extension region 16, and the fourth extension region 17 take the support rods 121 of the cutting portion 11 as proximal boundaries. But the support rods 121 of the cutting portion 11 do not belong to the support rods 121 in the first extension region 14, the second extension region 15, the third extension region 16, and the fourth extension region 17. That is, the cutting portion 11 is located outside the first extension region 14, the second extension region 15, the third extension region 16, and the fourth extension region 17. The distal end portion 111 of the cutting portion 11 is adjacent to the proximal end portion of the first extension region 14. The proximal end portion 112 of the cutting portion 11 is adjacent to the proximal end portion of the third extension region 16.

The hardness of the first extension region 14 is greater than the hardness of the second extension region 15, the hardness of the first extension region 14 is greater than the hardness of the fourth extension region 17, the hardness of the third extension region 16 is greater than the hardness of the second extension region 15, and the hardness of the third extension region 16 is greater than the hardness of the fourth extension region 17.

The first extension region 14 is directly or indirectly connected to the second extension region 15, and the first extension region 14 is directly or indirectly connected to the fourth extension region 17. When the first extension region 14 is indirectly connected to the second extension region 15, a transition region is provided between the first extension region 14 and the second extension region 15, and a hardness of the transition region is between the hardness of the first extension region 14 and the hardness of the second extension region 15. When the first extension region 14 is indirectly connected to the fourth extension region 17, another transition region is provided between the first extension region 14 and the fourth extension region 17, and a hardness of the another transition region is between the hardness of the first extension region 14 and the hardness of the fourth extension region 17.

When the cutting stent 10 of this embodiment is used for thrombectomy, since the hardness of the first extension region 14 is greater than the hardness of the second extension region 15 and the fourth extension region 17, during the process of the cutting stent 10 gradually entering a blood vessel with a smaller inner diameter, the support rods 121 in the second extension region 15 and the fourth extension region 17 undergo a larger circumferential shrinkage deformation than the support rods 121 in the first extension region 14, thereby reducing or avoiding excessive accumulation of the support rods 121 in the first extension region 14. Moreover, since the distal end portion 111 of the cutting portion 11 is adjacent to the proximal end portion of the first extension region 14, reducing or avoiding excessive accumulation of the support rods 121 in the first extension region 14 can reduce or avoid inward folding of the support rods 121 near the distal end portion 111 of the cutting portion 11 due to excessive accumulation, so as to improve the wall adherence of the cutting portion 11 in the blood vessel.

In addition, the hardness of the third extension region 16 is greater than the hardness of the second extension region 15 and the hardness of the fourth extension region 17, so that the third extension region 16 has a relatively high hardness, thereby enabling the third extension region 16 to have a relatively high axial tensile strength. When the cutting stent 10 is driven by the push-pull tube 20 to move in the blood vessel, the axial stretching amplitude of the third extension region 16 can be reduced, thereby reducing the inward shrinkage deformation amplitude caused by axial stretching of the cutting portion 11, and further avoiding separation of the cutting portion 11 from the blood vessel wall. Therefore, the hardness of the first extension region 14 being greater than the hardness of the second extension region 15 and the hardness of the fourth extension region 17, and the hardness of the third extension region 16 being greater than the hardness of the second extension region 15 and the hardness of the fourth extension region 17 can prevent excessive accumulation of the support rods 121 near the distal end portion 111 of the cutting portion 11, and also reduce the axial stretching amplitude of the cutting portion 11, so as to improve the wall adherence of the cutting portion 11.

The hardness of each region can be set by setting a rod width of each of the support rods 121 in each region. Under the same other conditions, the wider the rod width of the support rods 121 in a region, the higher the hardness of the region, and vice versa.

In an embodiment, the rod width of each of the support rods 121 in the first extension region 14 is greater than the rod width of each of the support rods 121 in the second extension region 15 and the fourth extension region 17, so that the hardness of the first extension region 14 is greater than the hardness of the second extension region 15 and the hardness of the fourth extension region 17, so as to avoid excessive concentration of the support rods 121 at the distal end portion 111 during the process of the cutting stent 10 entering a blood vessel with a smaller inner diameter.

In an embodiment, the rod width of each of the support rods 121 in the third extension region 16 is greater than the rod width of each of the support rods 121 in the second extension region 15 and the fourth extension region 17, so that the hardness of the third extension region 16 is greater than the hardness of the second extension region 15 and the hardness of the fourth extension region 17, thereby enabling the support strength of the third extension region 16 to be greater than the support strength of the second extension region 15 and the fourth extension region 17, so as to reduce inward shrinkage deformation caused by axial stretching of the cutting portion 11 and further avoid separation of the cutting portion 11 from the blood vessel wall.

The third extension region 16 is directly or indirectly connected to the second extension region 15, and the first extension region 14 is directly or indirectly connected to the fourth extension region 17. When the third extension region 16 is indirectly connected to the second extension region 15, a transition region is provided between the third extension region 16 and the second extension region 15. A hardness of the transition region is between the hardness of the third extension region 16 and the hardness of the second extension region 15.

In an embodiment, the hardness of the third extension region 16 is the same as the hardness of the first extension region 14.

In the embodiment shown in FIG. 7A, in a three-dimensional state, the second extension region 15 and the fourth extension region 17 are radially opposite to each other, and the hardness of the second extension region 15 is equal to the hardness of the fourth extension region 17. When the cutting stent 10 enters a blood vessel with a smaller inner diameter, the circumferential shrinkage amplitudes of the second extension region 15 and the fourth extension region 17 can be the same or substantially the same, thereby preventing the cutting stent 10 from shifting to a side with a larger shrinkage amplitude (e.g., a side where the second extension region 15 or the fourth extension region 17 is located in the circumferential direction) when entering a blood vessel with a smaller inner diameter due to different shrinkage amplitudes of the second extension region 15 and the fourth extension region 17, and further preventing the cutting portion 11 from being driven to shift synchronously and separate from the blood vessel wall.

Referring to FIG. 6, the second stent B is tapered. A smaller-diameter end of the second stent B is located at a distal side. A larger-diameter end of the second stent B is located at a proximal side. The larger-diameter proximal end of the second stent B is connected to the distal end of the second axial section A2. A radial dimension of the second stent B gradually decreases from the proximal end to the distal end. A radial dimension of a portion of the second stent B other than a portion connected to the first stent A is smaller than a radial dimension of the first stent A. The second stent B has a tapered inner cavity. A larger-diameter end of the second stent B is an open end. A smaller-diameter end of the second stent B is a closed end. The closed end is a closed end of the cutting stent 10.

When the cutting stent 10 is driven to move proximally in the blood vessel for thrombectomy, since the second stent B is tapered, the radial dimension of the second stent B gradually decreases from proximal to distal, and the radial dimension of the portion of the second stent B except the portion connected to the first stent A is smaller than the radial dimension of the first stent A, so that a radial outward pressing force of the second stent B on the blood vessel wall is smaller than that of the first stent A on the blood vessel wall, which helps reduce resistance between the cutting stent 10 and the inner wall of the blood vessel, thereby reducing a driving force required to drive the cutting stent 10 to move in the blood vessel. A smaller driving force results in a smaller axial stretching amplitude of the first axial section A1, thereby improving the wall adherence of the cutting portion 11.

Referring to FIGS. 6 and 7A together, a distal end of the third extension region 16 extends to a position near a boundary between the first stent A and the second stent B. The support rods 121 at the distal end of the third extension region 16 are directly connected to the support rods 121 at the distal end of the first stent A that are directly connected to the second stent B, so that an axial length of the third extension region 16 is close to an axial length of the first stent A. The closer the axial length of the third extension region 16 with higher hardness to the axial length of the first stent A, the more favorable it is to improve the overall axial tensile property of the first stent A, thereby improving the wall adherence of the cutting portion 11.

Referring to FIGS. 7A, 7C and 7D together, in an embodiment, each of the second extension region 15 and the fourth extension region 17 includes a plurality of first extension rods 122 and a plurality of second extension rods 123. Each of the first extension rods 122 is constituted by connecting a plurality of support rods 121, and each of the second extension rods 123 is formed by at least one support rod 121.

A proximal end of the first extension rod 122 is closer to a straight line L passing through the proximal end portion 112 of the cutting portion 11 and a distal end portion 181 of the cutting stent 10 than a distal end thereof. A distal end of the second extension rod 123 is closer to the straight line L than a proximal end thereof. Proximal ends of some second extension rods 123 abut against the cutting portion 11, and distal ends of the second extension rods 123 whose proximal ends abut against the cutting portion 11 abut against the support rods 121 in the third extension region 16. A supporting effect of the second extension rods 123 on the cutting portion 11 is better than a supporting effect of the first extension rods 122 on the cutting portion 11.

In an embodiment, by setting the rod width of each of the second extension rods 123 to be greater than the rod width of each of the first extension rods 122, the support strength of each of the second extension rods 123 is greater than the support strength of each of the first extension rods 122. When the cutting stent 10 enters a blood vessel with a smaller inner diameter, the second extension rods 123 can provide a relatively large proximal supporting force for the cutting portion 11, which helps the cutting portion 11 resist a distal resistance applied by the blood vessel, thereby reducing gathering of the support rods 121 in the axial section where the cutting portion 11 is located toward the distal side, further reducing the axial stretching amplitude of the cutting portion 11 and avoiding accumulation of the support rods 121 near the distal end portion 111 of the cutting portion 11, so as to improve the wall adherence of the cutting portion 11. Moreover, proximal ends of some second extension rods 123 abut against the cutting portion 11, and distal ends of the second extension rods 123 abut against the support rods 121 in the third extension region 16, so the third extension region 16 serves as a supporting base of the second extension rods 123 abutting against the cutting portion 11. Therefore, setting the hardness of the third extension region 16 to be greater than the hardness of the second extension region 15 and the fourth extension region 17 enables stable support for the second extension rods 123, thereby helping the second extension rods 123 support the cutting portion 11 and reducing the possibility of separation of the cutting portion 11 from the inner wall of the blood vessel during thrombectomy.

Although the higher the overall hardness of the first stent A, the better the cutting portion 11 can attach to the inner wall of the blood vessel, since the cutting stent 10 needs to pass through a curved blood vessel during thrombectomy, if the hardness of the first stent A is too high, the radial outward pressing force of the first stent A on the inner wall of the blood vessel will be too large, resulting in excessive stimulation of the inner wall of the blood vessel by the first stent A. In addition, an excessively high hardness of the first stent A is not conducive to the cutting stent 10 passing through a curved blood vessel.

Referring to FIGS. 7A and 7C together, in an embodiment, a part of the first extension rods 122 and the second extension rods 123 are located at a distal end of the first extension region 14. The first extension rods 122 located at the distal end of the first extension region 14 in the second extension region 15 and the fourth extension region 17 are radially opposite, and distal ends of two radially opposite ones of the first extension rods 122 are connected. The second extension rods 123 located at the distal end of the first extension region 14 in the second extension region 15 and the fourth extension region 17 are radially opposite, and distal ends of two radially opposite ones of the second extension rods 123 are connected. In this way, a section between the distal end of the first extension region 14 and the distal end of the second axial section A2 has greater flexibility than the axial section where the first extension region 14 is located, thereby reducing stimulation of the inner wall of the blood vessel by the section between the distal end of the first extension region 14 and the distal end of the second axial section A2, and facilitating the cutting stent 10 to pass through a curved blood vessel.

Referring to FIG. 8, in an embodiment, the third extension region 16 includes a proximal section 161 and a distal section 162 connected to each other. A proximal end of the proximal section 161 is directly connected to the cutting portion 11, and a distal end of the proximal section 161 is connected to a proximal end of the distal section 162. In FIG. 8, the proximal section 161 and the distal section 162 are separated by a dashed line X. A maximum circumferential width of the proximal section 161 is greater than a maximum circumferential width of the distal section 162.

Since the number of the support rods 121 in the first axial section A1 gradually increases from proximal to distal, the axial tensile strength of the first axial section A1 gradually increases from proximal to distal, so the axial tensile strength of a region of the first axial section A1 closer to the proximal end portion 112 is weaker. Therefore, when an area of a region with higher hardness of the cutting stent 10 is fixed, the closer the region with higher hardness of the cutting stent 10 to the proximal end portion 112 in the axial direction, the more favorable it is to improve the overall axial tensile strength of the first axial section A1; when an axial relative position relationship between the region with higher hardness and the proximal end portion 112 is fixed, the larger the area of the region with higher hardness, the more favorable it is to improve the overall axial tensile strength of the first axial section A1.

In this embodiment, the proximal end of the proximal section 161 is directly connected to the cutting portion 11, the maximum circumferential width of the proximal section 161 is greater than the maximum circumferential width of the distal section 162, and the hardness of the proximal section 161 of the third extension region 16 is higher than the hardness of the second extension region 15 and the fourth extension region 17, which helps improve the hardness in a region close to the proximal end portion 112 in the axial direction and increase the area of the region with higher hardness close to the proximal end portion 112, thereby improving the axial tensile strength of the first axial section A1 and further improving the wall adherence of the cutting portion 11.

Continuing to refer to FIG. 8, in an embodiment, the support rods 121 at the maximum circumferential width of the proximal section 161 are directly connected to the support rods 121 of the cutting portion 11. Compared with the support rods 121 in the second extension region 15 and the fourth extension region 17, the support rods 121 in the proximal section 161 are harder. The support rods 121 at the maximum circumferential width of the proximal section 161 are directly connected to the support rods 121 of the cutting portion 11, which helps improve the radial support property of the cutting portion 11, thereby improving the wall adherence of the cutting portion 11.

The support strength of each of the support rods 121 of the cutting portion 11 is greater than the support strength of the first extension rods 122 and the support strength of the second extension rods 123. For example, by setting the rod width of each of the support rods 121 of the cutting portion 11 to be greater than the rod width of each of the first extension rods 122 and the second extension rods 123, the support strength of each of the support rods 121 of the cutting portion 11 is greater than both the support strength of each of the first extension rods 122 and the support strength of each of the second extension rods 123, which can improve the radial supporting force of the cutting portion 11 and facilitate improving the wall adherence of the cutting portion 11.

Referring to FIGS. 3 and 4, the thrombectomy device 1 further includes an inner tube 60. The inner tube 60 is slidably inserted through the push-pull tube 20, and a distal end thereof extends out of the distal end of the push-pull tube 20 to be fixedly connected to a distal end of the second stent B. A driving force is applied to the inner tube 60 to slide the inner tube 60 distally relative to the push-pull tube 20, and the inner tube 60 cooperates with the push-pull tube 20 to apply an axial pulling force to both ends of the cutting stent 10, thereby reducing the radial dimension of the cutting stent 10.

Referring to FIGS. 3 and 4, the thrombectomy device 1 further includes a filter membrane 70. The filter membrane 70 is located in the inner cavity of the cutting stent 10, and is a bag-shaped structure having an opening portion 71 at a proximal end and a closed end at a distal end. The opening portion 71 is fixedly connected to a distal end of the first stent A (see FIG. 5) (e.g., fixed by suture). The thrombus scraped by the cutting portion 11 enters the inner cavity of the filter membrane 70 after entering the inner cavity of the cutting stent 10 to be intercepted, so that the thrombus can be prevented from escaping downstream.

The filter membrane 70 is flexible, and a portion of the filter membrane 70 located in the second stent B can protrude outward from the grid units 12 of the second stent B under a radial outward pressing force.

After thrombus scraping is completed, the thrombus collected by the cutting stent 10 needs to be transported to the outside of the body through a delivery channel established by an outer sheath for cleaning. An axial pulling force toward the outer sheath is applied to the cutting stent 10 to drive the cutting stent 10 carrying the thrombus into the inner cavity of the outer sheath.

Referring to FIGS. 3 and 4, the thrombectomy device 1 further includes an intermediate tube 80. The intermediate tube 80 is slidably sleeved on the inner tube 60 and located between the push-pull tube 20 and the inner tube 60. A distal end of the intermediate tube 80 is connected to a distal end of the filter membrane 70, and a proximal end of the intermediate tube 80 extends axially beyond the proximal end of the intermediate tube 80, so as to apply a driving force to the intermediate tube 80 to drive the distal end of the filter membrane 70 to move. When an axial position of the distal end of the filter membrane 70 needs to be adjusted, an axial force is applied to the proximal end of the intermediate tube 80 to drive the intermediate tube 80 to move axially, so that the distal end of the filter membrane 70 is driven by the intermediate tube 80 to move axially between a proximal position and a distal position. When the distal end of the filter membrane 70 is driven to the proximal position, the distal end of the filter membrane 70 is located outside the cutting stent 10, and an inner surface and an outer surface of the filter membrane 70 are reversed to facilitate dumping of the thrombus in the filter membrane 70; when the distal end of the filter membrane 70 is located at the distal position, the distal end of the filter membrane 70 is located in the inner cavity of the cutting stent 10 and can intercept the thrombus.

The technical features of the above embodiments can be combined arbitrarily. For the sake of brevity, all possible combinations of the technical features of the above embodiments are not described. However, as long as there is no contradiction in the combination of these technical features, they shall be considered as falling within the scope recorded in this description.

The above disclosures are merely preferred embodiments of the present disclosure, which certainly cannot be used to limit the scope of the claims of the present disclosure. Therefore, equivalent changes made according to the claims of the present disclosure shall still fall within the scope covered by the present disclosure.

## Claims

1. A thrombectomy device, **characterized by** comprising a cutting stent having an inner cavity; wherein the cutting stent is a grid structure with a plurality of grid units; wherein each of the grid units comprises a plurality of support rods connected end to end, the cutting stent comprises a cutting portion at a proximal end, the cutting portion is an opening communicating with the inner cavity, and the cutting portion comprises a proximal end portion and a distal end portion; wherein the cutting stent comprises a first extension region, a second extension region, a third extension region, and a fourth extension region extending in an axial direction; wherein the first extension region, the second extension region, the third extension region, and the fourth extension region are sequentially arranged and connected in a circumferential direction; wherein the cutting portion is connected to proximal ends of the first extension region, the second extension region, the third extension region, and the fourth extension region; wherein the distal end portion of the cutting portion is adjacent to a proximal end portion of the first extension region, the proximal end portion of the cutting portion is adjacent to a proximal end portion of the third extension region, the first extension region and the third extension region are radially opposite, and the second extension region and the fourth extension region are radially opposite; and wherein a hardness of the first extension region is greater than a hardness of the second extension region, a hardness of the first extension region is greater than a hardness of the fourth extension region, a hardness of the third extension region is greater than a hardness of the second extension region, and a hardness of the third extension region is greater than a hardness of the fourth extension region.

2. The thrombectomy device according to claim 1, **characterized in that** the third extension region comprises a proximal section and a distal section connected to each other, a proximal end of the proximal section is directly connected to the cutting portion, a distal end of the proximal section is connected to a proximal end of the distal section, and a maximum circumferential width of the proximal section is greater than a maximum circumferential width of the distal section.

3. The thrombectomy device according to claim 2, **characterized in that** the support rods at a position of the maximum circumferential width of the proximal section are directly connected to the cutting portion.

4. The thrombectomy device according to claim 1, **characterized in that** a rod width of each of the support rods in the first extension region is greater than a rod width of each of the support rods in the second extension region, and a rod width of each of the support rods in the first extension region is greater than a rod width of each of the support rods in the fourth extension region; a rod width of each of the support rods in the third extension region is greater than a rod width of each of the support rods in the second extension region, and a rod width of each of the support rods in the third extension region is greater than a rod width of each of the support rods in the fourth extension region.

5. The thrombectomy device according to claim 1, **characterized in that** the hardness of the second extension region is equal to the hardness of the fourth extension region.

6. The thrombectomy device according to claim 4 or 5, **characterized in that** each of the second extension region and the fourth extension region comprises one or more first extension rods and one or more second extension rods; each of the first extension rods is constituted by connecting a plurality of support rods, and each of the second extension rods is constituted by connecting at least one support rod; a proximal end of each of the first extension rods is closer to a straight line passing through the proximal end portion of the cutting portion and a distal end portion of the cutting stent than a distal end thereof, and a distal end of the second extension rod is closer to the straight line than a proximal end thereof; proximal ends of a part of the second extension rods abut against the cutting portion, and distal ends of the second extension rods whose proximal ends abut against the cutting portion abut against the support rods in the third extension region; a support strength of each of the second extension rods is greater than a support strength of each of the first extension rods.

7. The thrombectomy device according to claim 6, **characterized in that** a part of the first extension rods and a part of the second extension rods are located at a distal end of the first extension region, the first extension rods located at the distal end of the first extension region in the second extension region and the fourth extension region are radially opposite, distal ends of two radially opposite ones of the first extension rods are connected, the second extension rods located at the distal end of the first extension region in the second extension region and the fourth extension region are radially opposite, and distal ends of two radially opposite ones of the second extension rods are connected.

8. The thrombectomy device according to claim 6, **characterized in that** a support strength of the cutting portion is greater than both the support strength of the first extension rod and the support strength of the second extension rod.

9. The thrombectomy device according to claim 1, **characterized in that** the cutting stent comprises a first stent and a second stent, both the first stent and the second stent are parts of the grid structure, the first stent and the second stent are connected to each other and jointly define the inner cavity of the cutting stent, a circumferentially closed edge of a proximal end of the first stent constitutes the cutting portion, a distal end of the third extension region extends to a position near a boundary between the first stent and the second stent, and the support rods at the distal end of the third extension region are directly connected to the support rods at a distal end of the first stent that are directly connected to the second stent.

10. The thrombectomy device according to claim 1, **characterized in that** the cutting stent comprises a first stent and a second stent, both the first stent and the second stent are parts of the grid structure, the first stent and the second stent are connected to each other and jointly define the inner cavity of the cutting stent, a circumferentially closed edge of a proximal end of the first stent constitutes the cutting portion, a radial dimension of the second stent gradually decreases from the proximal end to the distal end, and a radial dimension of a portion of the second stent other than a portion connected to the first stent is smaller than a radial dimension of the first stent.
